(19) **Europäisches Patentamt**
**European Patent Office**
**Office européen des brevets**

(11) **EP 4 162 252 B1**

(12) **EUROPÄISCHE PATENTSCHRIFT**

(45) Veröffentlichungstag und Bekanntmachung des Hinweises auf die Patenterteilung:
**13.08.2025  Patentblatt 2025/33**

(21) Anmeldenummer: **20768545.4**

(22) Anmeldetag: **04.09.2020**

(51) Internationale Patentklassifikation (IPC):
**G01N 15/02** (2024.01)      **G01N 22/00** (2006.01)
**G01N 33/15** (2006.01)

(52) Gemeinsame Patentklassifikation (CPC):
**G01N 15/0205; G01N 22/00; G01N 33/15**

(86) Internationale Anmeldenummer:
**PCT/EP2020/074756**

(87) Internationale Veröffentlichungsnummer:
**WO 2021/244768 (09.12.2021 Gazette 2021/49)**

(54) **VERFAHREN ZUR BESTIMMUNG VON MINDESTENS EINER KENNGRÖSSE EINER PARTIKELGRÖSSENVERTEILUNG SOWIE EINE VORRICHTUNG MIT EINER MESSEINRICHTUNG**

METHOD FOR DETERMINING AT LEAST ONE CHARACTERISTIC VALUE OF A PARTICLE SIZE DISTRIBUTION AND A DEVICE WITH A MEASURING DEVICE

PROCEDE POUR DETERMINER AU MOINS UNE VALEUR CARACTERISTIQUE D'UNE DISTRIBUTION DE TAILLE DE PARTICULES ET D'UN DISPOSITIF AVEC UN DISPOSITIF DE MESURE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.06.2020  DE 102020114726**

(43) Veröffentlichungstag der Anmeldung:
**12.04.2023  Patentblatt 2023/15**

(73) Patentinhaber: **TEWS Elektronik GmbH & Co. KG**
**22459 Hamburg (DE)**

(72) Erfinder:
• **SCHLEMM, Udo**
**22607 Hamburg (DE)**
• **RICHTER, Hendrik**
**20255 Hamburg (DE)**

(74) Vertreter: **Hauck Patent- und Rechtsanwälte PartmbB**
**Postfach 11 31 53**
**20431 Hamburg (DE)**

(56) Entgegenhaltungen:
**JP-B2- 4 230 058**

• **CLARK NICHOLAS ET AL: "Particle size characterisation of metals powders for Additive Manufacturing using a microwave sensor", POWDER TECHNOLOGY, vol. 327, 2 December 2017 (2017-12-02), Basel (CH), pages 536 - 543, XP055774728, ISSN: 0032-5910, DOI: 10.1016/j.powtec.2017.11.042**
• **AMIR MOSTAFAEI ET AL: "Comparison of characterization methods for differently atomized nickel-based alloy 625 powders", POWDER TECHNOLOGY, vol. 333, 7 April 2018 (2018-04-07), Basel (CH), pages 180 - 192, XP055669379, ISSN: 0032-5910, DOI: 10.1016/j.powtec.2018.04.014**

**Beschreibung**

Die vorliegende Erfindung betrifft ein Verfahren zur Bestimmung von mindestens einer Kenngröße einer Partikelgrößenverteilung in einem bewegten Teilchenstrom. Ebenfalls betrifft die Erfindung eine Vorrichtung zur Erzeugung eines bewegten Teilchenstroms mit einer Messeinrichtung zur Bestimmung von mindestens einer Kenngröße einer Partikelgrößenverteilung in dem bewegten Teilchenstrom.

Aus WO 2009/030314 ist ein Verfahren zur Messung eines Feuchtewerts von dielektrischen Stoffen unter Verwendung von mindestens einem Resonator bekannt. Hierbei werden für mindestens zwei Resonanzmoden mit voneinander verschiedenen Resonanzfrequenzen jeweils eine Verschiebung der Resonanzfrequenz ausgewertet und aus der gemessenen Verschiebung der Resonanzfrequenz ein dichteunabhängiger Feuchtewert berechnet. Der besondere Vorteil dieses Verfahrens soll darin liegen, dass bei der Bestimmung des Feuchtewerts nicht länger auf einen Dämpfungswert als Maß für die Feuchte zurückgegriffen werden muss. Vielmehr wird aus mindestens zwei bei unterschiedlichen Resonanzfrequenzen auftretenden Verschiebungen der Resonanzfrequenz mit großer Zuverlässigkeit ein dichteunabhängiger Feuchtewert berechnet. Mit dem Zurückgreifen auf die Frequenzverschiebung bei zwei Resonanzfrequenzen kann unter Verzicht auf Dämpfungskennwerte eine Partikelgröße D berechnet werden. In der Praxis hat sich herausgestellt, dass die Bestimmung einer Partikelgröße D in einem bewegten Teilchenstrom nur sehr unzuverlässig möglich ist.

Aus DE 101 11 833 C1 ist eine Messsonde zur In-line-Bestimmung der Größe von bewegten Partikeln in transparenten Medien bekannt geworden. Die Messsonde besitzt einen rohrförmigen Messsondenkörper, in den einzelne von den bewegten Partikeln eintreten und dort optisch vermessen werden. Hierzu werden die Partikel unter Verwendung eines Disperigiermediums vereinzelt.

Aus DE 3 241 544 A1 ist ein Verfahren zum Überwachen und/oder Steuern bei Trocknungs-, Granulier-, Instanziier-, Dragier- und Film-Coating-Prozessen bekannt geworden. Bei dem bekannten Verfahren wird die Feuchte der Abluft sowie die Feuchte der Zuluft gemessen und die sich daraus ergebende Feuchtedifferenz zur Steuerung des Arbeitsprozesses eingesetzt.

Aus JP 4230058 B2 ist bekannt, einen Quantil-Wert mit Hilfe eines neuronalen Netzes zu bestimmen, wobei die Messung des Materials optisch erfolgt und das neuronale Netz mit einer Teilchengrößenverteilung trainiert wird.

Clark et. al. offenbart in "Particle size characterisation of metals powders for Additive Manufacturing using a microwave sensor" (POWDER TECHNOLOGY, Bd. 327, 2. Dezember 2017 (2017-12-02), Seiten 536-543) den Oberbegriff von Anspruch 1.

Der Erfindung liegt die Aufgabe zugrunde ein Verfahren, um mindestens eine Kenngröße einer Partikelgrößenverteilung in einem bewegten Teilchenstrom zuverlässig zu messen. Ebenso liegt der Erfindung die Aufgabe zugrunde für eine Vorrichtung zur Erzeugung eines bewegten Teilchenstroms eine solche Messeinrichtung bereitzustellen.

Erfindungsgemäß wird die Aufgabe durch ein Verfahren mit den Merkmalen nach Anspruch 1 und eine Vorrichtung mit den Merkmalen nach Anspruch 9 gelöst. Vorteilhafte Weiterbildungen bilden die Gegenstände der Unteransprüche.

Das erfindungsgemäße Verfahren ist dafür vorgesehen, mindestens eine Kenngröße einer Partikelgrößenverteilung in einem bewegten Teilchenstrom zu bestimmen. Für die Bestimmung wird mindestens ein Mikrowellenresonator verwendet, der jeweils mindestens zwei Messwerte für den Teilchenstrom bereitstellt. Ein wichtiges Merkmal ist dabei, dass mit der Kenngröße ein Quantil der Partikelgrößenverteilung bestimmt wird. Anders als im Stand der Technik, bei dem beispielsweise eine mittlere Partikelgröße oder auch eine andere mittlere Größe bestimmt wurde, wird erfindungsgemäß durch das Quantil ein Anteil an einer Verteilung bestimmt, d. h. es werden beispielsweise die Anzahl, die Masse oder eine sonstige Größe dahingehend ausgewertet, wie groß der Anteil der Teilchen ist, die kleiner oder gleich dem Wert des Quantils sind. Es wird also nicht auf einen Größenwert selber, sondern auf einen Anteil bis zu dem Größenwert abgestellt. So definiert das Quantil einen Schwellwert, bei dem ein bestimmter Anteil der Werte kleiner als das Quantil ist, während der Rest größer ist. Das 25 % Quantil bezeichnet beispielsweise den Wert, für den gilt, dass 25 % aller Werte kleiner sind als dieser Wert und 75 % größer. Ein besonderer Schritt, Partikelgrößenverteilungen unter Verwendung eines Mikrowellenresonators erfindungsgemäß auszuwerten besteht darin, nicht auf bestimmte Werte oder Durchschnittswerte zu schauen, sondern die gemessenen Größen dahingehend auszuwerten, dass stets auch die Beiträge aller bewegten Teilchen berücksichtigt werden, die kleiner als das zu untersuchende Quantil ist. Neben der Verwendung von mehreren Mikrowellenresonatoren ist es auch möglich, Mikrowellenresonatoren zu verwenden, die zwei oder mehr Resonanzmoden aufweisen. Für jede Resonanzmode können dann jeweils zwei Messwerte für den Teilchenstrom bereitgestellt werden.

In einer bevorzugten Ausgestaltung hat es sich als besonders vorteilhaft herausgestellt, als die beiden Messwerte des Mikrowellenresonators auf eine Resonanzfrequenzverschiebung und eine Resonanzkurvenverbreiterung abzustellen. Die Resonanzfrequenzverschiebung und die Resonanzkurvenverbreiterung (B) als Messwert sind grundsätzlich dichtabhängige Größen, während der Quotient der beiden Messwerte eine masse- bzw. dichteunabhängige Größe liefert. Insbesondere durch das Abstellen auf das Quantil ist die Verwendung von beiden Messwerten des Mikrowellenresonators mit Resonanzfrequenzverschiebung und Resonanzkurvenverbreiterung besonders vorteilhaft. Statt der Resonanzkurvenverbreiterung können auch andere Messwerte des Mikrowellenresonators verwendet werden, die Auskunft über die Dämpfung der Resonanz geben.

In einer bevorzugten Weiterbildung des Verfahrens wird mindestens eine Temperatur des Teilchenstroms ausgewertet. Die Temperatur des Teilchenstroms ergibt sich aus der Temperatur der zugeführten Luft und der Verdampfungswärme. Die Temperatur eines feuchten Teilchenstroms ist also aufgrund der stärkeren Verdampfung bei konstanter Zulufttemperatur niedriger als die eines trockenen Teilchenstroms (bei kontanter Füll- und Zuluftmenge).

Für einen bewegten Teilchenstrom in einer Wirbelschicht werden bevorzugt als weitere Messgrößen mindestens eine der folgenden Größen ausgewertet: Zuluftmenge und Füllmenge der Wirbelschicht. Die Zuluftmenge wird an Wirbelschichttrocknern in Abhängigkeit vom jeweiligen Prozess eingestellt und normalerweise auch während der Prozesse variiert. Sie kann beispielsweise in Kubikmeter/Stunde [m^3/h] als die Luftmenge angegeben werden. Die Füllmenge gibt in einer Wirbelschichtanlage beispielsweise an, wie viel Kilogramm [kg] an Material sich in der Wirbelschichtanlage finden.

Bevorzugt hat sich herausgestellt, dass das mindestens eine Quantil sehr genau durch eine lineare Approximation der ausgewerteten Messgrößen bestimmt werden kann. Dies bedeutet die verwendeten Messgrößen gehen als einfache Linearkombination zusätzlich mit einem konstanten Term in die Bestimmung des Quantils ein. Diese lineare Approximation macht auch deutlich, dass für die Mikrowellenmesswerte die Bestimmung des Quantils die geeigneten Kenngrößen der Partikelgrößenverteilung sind. Selbstverständlich können aus diesen Größen weitere Größen wie mittleres Teilchengewicht oder mittlerer Teilchendurchmesser bestimmt werden. Entscheidend ist aber, dass primär das Quantil ermittelt wird.

Bevorzugt ist es möglich, verschiedene Partikelgrößenverteilungen zu betrachten.

Einerseits es möglich, auf eine Anzahlverteilungssumme, eine Längenverteilungssumme, eine Flächenverteilungssumme oder eine Volumen-/Massenverteilungssumme abzustellen. Von besonderem Interesse für ein gutes Verständnis der Partikelgrößenverteilung ist es, mehrere Größen gleichzeitig zu bestimmen. Beispielsweise könnte mit den gleichen Messwerten, aber unterschiedlichen Koeffizienten bei der Linearkombination Anzahl-Verteilungssumme und eine Volumen-Verteilungssumme betrachtet werden. Es ist auch möglich, mehrere Quantile einer Verteilungssumme zu bestimmen.

Die erfindungsgemäße Aufgabe wird ebenfalls durch eine Vorrichtung zur Erzeugung eines bewegten Teilchenstroms mit den Merkmalen aus Anspruch 9 gelöst. Die Vorrichtung besitzt eine Messeinrichtung zur Bestimmung von mindestens einer Kenngröße einer Partikelgrößenverteilung in dem bewegten Teilchenstrom auf. Die Messeinrichtung weist mindestens einen Mikrowellenresonator auf, der jeweils mindestens zwei Messwerte für den Teilchenstrom bereitstellt. Die mindestens zwei Messwerte sind bevorzugt die Resonanzfrequenzverschiebung und eine Resonanzkurvenverbreiterung. Ferner ist die Messeinrichtung dazu eingerichtet, aus den beiden Messwerten des Mikrowellenresonators mindestens ein Quantil der Partikelgrößenverteilung auszuwerten. Die Messeinrichtung, an der die Messwerte des mindestens einen Mikrowellenresonators anliegen, kann räumlich zusammen mit dem Mikrowellenresonator angeordnet sein oder auch von diesem getrennt. Der Mikrowellenresonator kann dazu vorgesehen sein, zwei oder mehr Resonanzmoden zu erzeugen, wobei in jeder der Resonanzmoden die mindestens beiden Messwerte erfasst werden können.

Weiter bevorzugt ist die Messeinrichtung dazu eingerichtet, zusätzlich eine Temperatur des Teilchenstroms auszuwerten.

Bevorzugt ist die Messeinrichtung derart angeordnet, dass die Messgrößen in einer Wirbelschicht gemessen werden. Insbesondere bei dem Einsatz in einer Wirbelschicht ist die Messeinrichtung dazu eingerichtet, mindestens eine der folgenden Größen wie Zuluft- und Füllmenge der Wirbelschicht mit auszuwerten. Die Zuluft- und die Füllmenge beeinflussen die Mikrowellenmessung stark und werden deshalb bevorzugt für die Bestimmung des zu bestimmenden Quantils eines Teilchenstroms berücksichtigt.

Die Messeinrichtung ist bevorzugt so ausgerichtet, dass das Quantil für eine Anzahl-Verteilungssumme, eine Längen-Verteilungssumme, eine Flächen-Verteilungssumme oder eine Volumenverteilungssumme und/oder eine Massenverteilungssumme bestimmt werden. Wichtig hierbei ist, dass die Messeinrichtung auch mehrere Quantile gleichzeitig bestimmen kann.

Bei einer Erfassung von mehreren Quantilen über der Zeit, kann so die zuverlässige Bestimmung der in der bewegten Schicht ablaufenden Prozesse gewonnen werden.

Die obige Erfindung wird nachfolgend mithilfe von einigen Messwerten näher erläutert. Es zeigen:

Figur 1    die zeitliche Entwicklung von drei Feinheitsmerkmalen die sich auf die Anzahl-Verteilungssumme beziehen,

Figur 2    drei Feinheitsmerkmale, die sich auf die Volumen-Verteilungssumme beziehen und

Figur 3    die zeitliche Entwicklung für einen mittleren Durchmesser der Teilchen.

Wirbelschichtprozesse werden in zahlreichen unterschiedlichen technischen Feldern eingesetzt. Ein wichtiges Einsatzgebiet ist der pharmazeutische Produktionsprozess, bei der Herstellung von diskreten Wirkstoffeinheiten, die beispielsweise zu Tabletten verpresst oder in Kapseln verfüllt werden. Hier wird ein Granulationsprozess mit einem anschließenden Wirbelschichttrocknungsprozess eingesetzt. Beim Granulationsprozess wird die vorliegende pharmazeutische Pulvermischung unter Einsprühen einer oftmals wässrigen Lösung zu einem Granulat mit definierter Partikelgröße verarbeitet. Beim anschließenden Wirbelschicht-Trocknungsprozess wird das Granulat bis zu einer definierten Zielfeuchte getrocknet. Beide Prozesse können in getrennten Anlagen erfolgen, es ist aber auch möglich, diese kombiniert in einer Anlage durchzuführen. Ein wichtiger Parameter zur Charakterisierung der Qualität des hergestellten Substrats ist neben dem Feuchtegehalt die mittlere Partikelgröße des Granulats. Neben der Überwachung des Prozesses und seines Endprodukts erlaubt die Messung der Partikelgrößenverteilung auch betriebliche Fehlfunktionen beispielsweise an den Sprühdüsen zu erkennen. Wichtig hierbei ist, sich vor Augen zu führen, dass nicht nur ein mittlerer Partikeldurchmesser ausschlaggebend ist, sondern Kenntnisse der gesamten Partikelgrößenverteilung hilfreich sind, um den Prozess zu beurteilen. Liegen beispielsweise große Partikel vor, sogenanntes "Überkorn" führt dies nicht unbedingt zu einer relevanten Erhöhung des durchschnittlichen Partikeldurchmessers, ist aber gleichwohl für weitere Verarbeitungsprozesse schädlich. Ebenso kann beispielsweise durch mechanische Belastung bei nicht ausreichender Stabilität des Granulats ein hoher Feinanteil entstehen, der ebenfalls für eine nachfolgende Weiterverarbeitung schwierig ist. Die Eigenschaften des Granulats mit seiner Partikelgrößenverteilung haben direkten Einfluss auf die nachfolgende Verarbeitung und auch auf die Eigenschaften beispielsweise der fertigen Tablette, im Hinblick auf ihre Auflösungskinetik und eine gleichförmige Abgabe des Wirkstoffgehalts.

Die Messung der Partikelgröße direkt im Wirbelschichtprozess erfolgt in der Zeit hauptsächlich mit einem Laserverfahren, wie es beispielsweise in DE 10 111 833 C1 beschrieben ist. Nachteilig bei dem optischen Verfahren ist, dass es äußerst empfindlich gegenüber Verschmutzungen ist und es zudem nur zur optischen Messung der Partikelgröße, nicht aber zur gleichzeitigen Messung des Feuchtegehalt geeignet ist.

Bei der Messung in einem Teilchenstrom kann zwischen dem Partikel (disperse Phase) und seinem umgebenden Medium (kontinuierliche Phase) unterschieden werden. Bei der Wirbelschicht bildet das trocknende Granulat die Partikel, während die umgebende Luft das kontinuierliche Medium bildet. Es ist üblich, Körner, Tropfen oder Blasen mithilfe eines zu messenden äquivalenten Durchmessers zu entscheiden und entsprechend ihrer Größe in ausgewählte Klassen einzuordnen. Zur Darstellung einer Partikelgrößenverteilung werden die Mengenanteile bestimmt, mit denen die jeweiligen Partikelklassen an der dispersen Phase beteiligt sind.

Es sind unterschiedliche Mengenarten bekannt: Werden die Partikel gezählt, so ist die Mengenart die Anzahl. Bei einer Wägung hingegen ist es die Masse bzw. bei homogener Dichte das Volumen. Weitere Mengenarten leiten sich aus Länge, Projektions- und Oberflächen her. Allgemein kann unterschieden werden:

| Mengenart | Index R |
|---|---|
| Anzahl | 0 |
| Länge | 1 |
| Fläche | 2 |
| Volumen (Masse) | 3 |

Es ist üblich, zur grafischen Darstellung auf ein normiertes Mengenmaß abzustellen, sodass die Abhängigkeit der Mengenanteile von der verwendeten Gesamtmenge eliminiert sind. Unter der Verwendung der vorstehend bezeichneten Indizes ergibt sich eine Anzahlverteilungssumme $Q_0$ und beispielsweise eine Volumenverteilungssumme $Q_3$. Bezeichnen wir mit X eine Partikelgröße als Äquivalentdurchmesser, so ergibt sich in der üblichen Notation beispielsweise $X_{10,0}$ für das Feinheitsmerkmal bei dem die Verteilungssumme $Q_0$ den Wert 10% annimmt. Anders ausgedrückt, das 10% Quantil der Verteilungsfunktion liegt bei dem Wert $X_{10,0}$, dies bedeutet 10% aller Teilchen haben diesen oder einen kleineren Durchmesser.

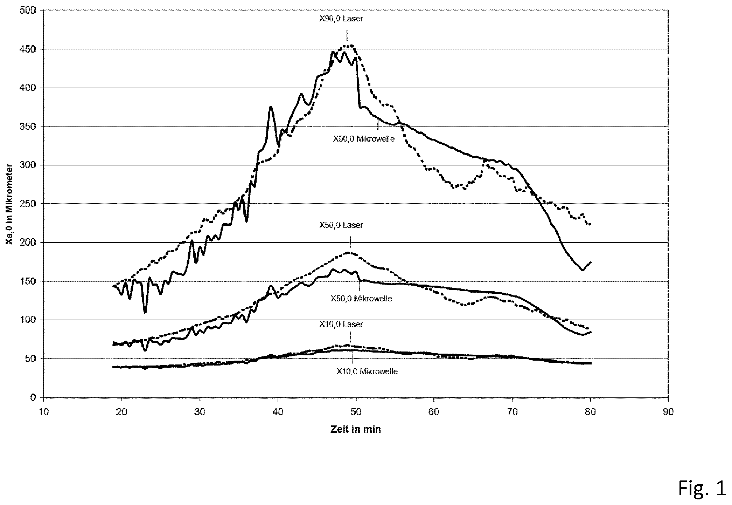

Betrachtet man Figur 1 so sind dort mit der durchgezogenen Linie die Messergebnisse des erfindungsgemäßen Verfahrens aufgetragen. Die obere Kurve $X_{90,0}$ ist während der Prozessdauer von 20-80 Minuten dargestellt. Die Ordinate zeigt den Durchmesser der Teilchen. Ein Wert von ungefähr 400 $\mu$m wie er beispielsweise kurz vor 50 Minuten und kurz nach 50 Minuten Prozessdauer auftritt in der Kurve $X_{90,0}$ bedeutet, dass 90% der Teilchen einen Durchmesser besitzen der kleiner oder gleich 400 $\mu$m ist. Die Kurve $X_{50,0}$ gibt den Äquivalentdurchmesser an, den 50% bezogen auf die Anzahl einen Durchmesser von beispielsweise weniger als 150 $\mu$m besitzen. Das Feinheitsmerkmal $X_{10,0}$ gibt den maximalen Durchmesser der 10% kleinsten Teilchen an. Die zeitliche Entwicklung dieser drei Feinheitsmerkmale gibt einen guten Aufschluss über die Korngrößenverteilung. Ist beispielsweise der Wert für $X_{10,0}$ zu klein, so kann daraus abgeleitet werden, dass 10% der Teilchen bezogen auf die Anzahl kleiner als dieser Wert sind und damit möglicherweise zu klein. Ebenso kann ein zu großer Wert für $X_{90,0}$ Hinweis darauf sein, dass ein Überkorn mit vereinzelten großen Körnern vorliegt.

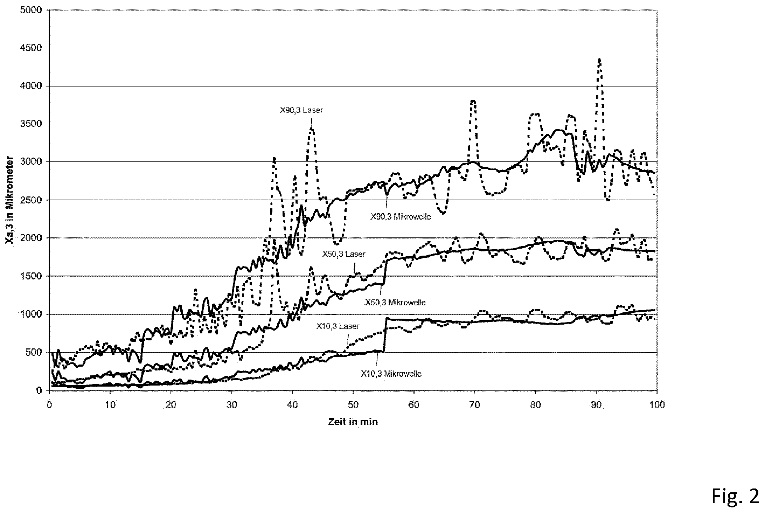

Figur 2 zeigt das Feinheitsmerkmal in $\mu$m bezogen auf die Volumenverteilungssumme. Die Kurve $X_{90,3}$ gibt die Quantile für die Volumenverteilungssumme an. Dies bedeutet $X_{90,3}$ bezeichnet beispielsweise den größten Durchmesser der Partikel, die 90% des Gesamtvolumens bilden.

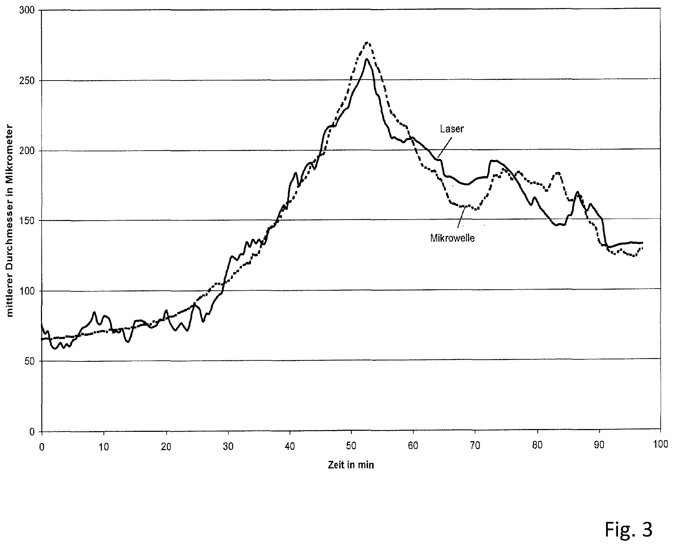

Figur 3 zeigt wie der mittlere Durchmesser der Teilchen sich mit der Zeit entwickelt Der mittlere Partikeldurchmesser nimmt während der Granulation stetig zu und nimmt während der Trocknungsphase durch die ständige Kollision der Partikel wieder ab. Der Übergang zwischen Granulation und Trocknungsphase erfolgt ungefähr bei 52 bis 55 min.

Figuren 1-3 zeigen jeweils parallele optische Messungen, die als Lasermessung bezeichnet sind. Der Vergleich zeigt, dass auch unter Verwendung eines Mikrowellenresonators hier zuverlässig Werte gewonnen werden können.

Für die Auswertung der Messwerte haben sich die folgenden Ansätze bewährt

$$X_{a,0} = a_1 \cdot A + a_2 \cdot B + a_3 \cdot L + a_4 \cdot T + a_5 \cdot F + a_0$$

$$X_{a,3} = b_1 \cdot A + b_2 \cdot B + b_3 \cdot L + b_4 \cdot T + b_5 \cdot F + b_0$$

wobei hier $X_{a,0}$ die Feinheitsmerkmale zu den Quantilen a der Anzahlverteilungssumme in $\mu m$ und $X_{a,3}$ die Feinheitsmerkmale zu den Quantilen a der Volumenverteilungssumme in $\mu m$ bezeichnen und $a_i$ und $b_i$ jeweils die Kalibrationskoeffizienten. Die zu wertenden Messgrößen sind A für die Resonanzfrequenzverschiebung einer Resonanzmode in MHz, B eine Verbreiterung der Resonanzkurve derselben Resonanzmode in MHz, L die Zuluftmenge zur Wirbelschicht in $m^3/h$, T die Produkttemperatur in Grad Celsius und F die Füllmenge der Wirbelschichtanlage in kg.

**Patentansprüche**

1. Verfahren zur Bestimmung von mindestens einer Kenngröße einer Partikelgrößenverteilung in einem bewegten Teilchenstrom unter Verwendung mindestens eines Mikrowellenresonators, der jeweils mindestens zwei Messwerte für den Teilchenstrom bereitstellt, **dadurch gekennzeichnet, dass** aus den Messwerten mindestens ein Quantil der Partikelgrößenverteilung bestimmt wird.

2. Verfahren nach Anspruch 1, **dadurch gekennzeichnet, dass** die mindestens zwei Messwerte des Mikrowellenresonators eine Resonanzfrequenzverschiebung (A) und eine Resonanzkurvenverbreiterung (B) betreffen.

3. Verfahren nach Anspruch 1 oder 2, **dadurch gekennzeichnet, dass** zusätzlich mindestens eine Temperatur des Teilchenstroms ausgewertet wird.

4. Verfahren nach einem der Ansprüche 1 bis 3, **dadurch gekennzeichnet, dass** der bewegte Teilchenstrom in einer Wirbelschicht vorliegt.

5. Verfahren nach Anspruch 4, **dadurch gekennzeichnet, dass** als weitere Messgröße mindestens eine der folgenden Größen ausgewertet wird: Zuluftmenge und Füllmenge der Wirbelschicht.

6. Verfahren nach einem der Ansprüche 1 bis 5, **dadurch gekennzeichnet, dass** das Quantil durch die ausgewerteten Messgrößen linear approximiert wird.

7. Verfahren nach einem der Ansprüche 1 bis 6, **dadurch gekennzeichnet, dass** das Quantil sich auf eine Anzahlverteilungssumme, eine Längenverteilungssumme, eine Flächenverteilungssumme oder eine Volumenverteilungssumme oder eine Massenverteilungssumme bezieht.

8. Verfahren nach einem der Ansprüche 1 bis 7, **dadurch gekennzeichnet, dass** mehrere Quantile über die Zeit erfasst werden.

9. Vorrichtung zur Erzeugung eines bewegten Teilchenstroms mit einer Messeinrichtung zur Bestimmung von mindestens einer Kenngröße einer Partikelgrößenverteilung in dem bewegten Teilchenstrom, die mindestens einen Mikrowellenresonator aufweist, der jeweils mindestens zwei Messwerte für den Teilchenstrom bereitstellt, **dadurch gekennzeichnet, dass** die Messeinrichtung dazu eingerichtet ist, aus den mindestens zwei Messwerten des Mikrowellenresonators mindestens ein Quantil der Partikelgrößenverteilung auszuwerten.

10. Vorrichtung nach Anspruch 9, **dadurch gekennzeichnet, dass** die Messeinrichtung derart angeordnet ist, dass zusätzlich eine Temperatur des Teilchenstroms gemessen wird.

11. Vorrichtung nach Anspruch 9 oder 10, **dadurch gekennzeichnet, dass** die Messeinrichtung derart angeordnet ist, dass die Kenngrößen in einer Wirbelschicht gemessen werden.

12. Vorrichtung nach Anspruch 11, **dadurch gekennzeichnet, dass** die Messeinrichtung eingerichtet ist, mindestens eine der folgenden Größen wie Zuluftmenge und Füllmenge der Wirbelschicht mit auszuwerten.

13. Vorrichtung nach Anspruch 11 oder 12, **dadurch gekennzeichnet, dass** die Messeinrichtung eingerichtet ist, das Quantil durch die ausgewerteten Messgrößen linear zu approximieren.

14. Vorrichtung nach einem der Ansprüche 9 bis 13, **dadurch gekennzeichnet, dass** die Messeinrichtung eingerichtet ist, das Quantil für eine Anzahlverteilungssumme, eine Längenverteilungssumme, eine Flächenverteilungssumme oder eine Volumenverteilungssumme oder eine Massenverteilungssumme zu bestimmen.

15. Vorrichtung nach einem der Ansprüche 9 bis 14, **dadurch gekennzeichnet, dass** die Messeinrichtung dazu eingerichtet ist, mehrere Quantile über die Zeit zu erfassen.

## Claims

1. Method for determining at least one parameter of a particle size distribution in a moving particle flow using at least one microwave resonator, which provides at least two measured values for the particle flow respectively, **characterized in that** at least one quantile of the particle size distribution is determined from the measured values.

2. Method according to claim 1, **characterized in that** the at least two measured values of the microwave resonator relate to a resonance frequency shift (A) and a resonance curve broadening (B).

3. Method according to claim 1 or 2, **characterized in that** at least one temperature of the particle flow is additionally evaluated.

4. Method according to one of claims 1 to 3, **characterized in that** the moving particle flow is present as a fluidized layer.

5. Method according to claim 4, **characterized in that** at least one of the following variables is evaluated as a further measured variable: supply air amount and filling amount of the fluidized layer.

6. Method according to one of claims 1 to 5, **characterized in that** the quantile is approximated linearly with the evaluated measured variables.

7. Method according to one of claims 1 to 6, **characterized in that** the quantile relates to a number distribution sum, a length distribution sum, an area distribution sum or a volume distribution sum or a mass distribution sum.

8. Method according to one of claims 1 to 7, **characterized in that** multiple quantiles are recorded over time.

9. Device for generating a moving particle flow with a measuring device for determining at least one parameter of a particle size distribution in the moving particle flow, which measuring device comprises at least one microwave resonator which provides at least two measured values for the particle flow respectively, **characterized in that** the measuring device is designed to evaluate at least one quantile of the particle size distribution from the at least two measured values of the microwave resonator.

10. Device according to claim 9, **characterized in that** the measuring device is arranged in such a way that a temperature of the particle flow is additionally measured.

11. Device according to claim 9 or 10, **characterized in that** the measuring device is arranged in such a way that the parameters are measured in a fluidized layer.

12. Device according to claim 11, **characterized in that** the measuring device is designed to evaluate at least one of the following variables, such as supply air amount and filling amount of the fluidized layer.

13. Device according to claim 11 or 12, **characterized in that** the measuring device is designed to approximate the quantile linearly with the evaluated measured variables.

14. Device according to one of claims 9 to 13, **characterized in that** the measuring device is designed to determine the quantile for a number distribution sum, a length distribution sum, an area distribution sum or a volume distribution sum or a mass distribution sum.

15. Device according to one of claims 9 to 14, **characterized in that** the measuring device is set up to record multiple quantiles over time.

## Revendications

1. Procédé de détermination d'au moins une grandeur caractéristique d'une distribution de taille de particules dans un flux particulaire en mouvement à l'aide d'au moins un résonateur à microondes, lequel fournit respectivement au moins deux valeurs de mesure pour le flux particulaire, **caractérisé en ce qu'**au moins un quantile de la distribution de taille de particules est déterminé à partir des valeurs de mesure.

2. Procédé selon la revendication 1, **caractérisé en ce que** les au moins deux valeurs de mesure du résonateur à microondes concernent un décalage de fréquence de résonance (A) et une propagation de courbe de résonance (B).

3. Procédé selon la revendication 1 ou 2, **caractérisé en ce qu'**au moins une température du flux particulaire est également évaluée.

4. Procédé selon l'une des revendications 1 à 3, **ca-**

**ractérisé en ce que** le flux particulaire en mouvement est présent dans une couche fluidisée.

5. Procédé selon la revendication 4, **caractérisé en ce que** l'une au moins des grandeurs suivantes est évaluée en tant que grandeur de mesure supplémentaire : quantité d'air alimenté et quantité de remplissage de la couche fluidisée.

6. Procédé selon l'une des revendications 1 à 5, **caractérisé en ce que** le quantile est approximé linéairement par les grandeurs de mesure évaluées.

7. Procédé selon l'une des revendications 1 à 6, **caractérisé en ce que** le quantile se rapporte à une somme de distribution numérique, à une somme de distribution des longueurs, à une somme de distribution des surfaces ou à une somme de distribution des volumes ou à une somme de distribution des masses.

8. Procédé selon l'une des revendications 1 à 7, **caractérisé en ce que** plusieurs quantiles sont détectés dans le temps.

9. Dispositif de production d'un flux particulaire en mouvement avec un moyen de mesure destiné à déterminer au moins une grandeur caractéristique d'une distribution de taille de particules dans le flux particulaire en mouvement, lequel présente respectivement au moins un résonateur à microondes fournissant au moins deux valeurs de mesure pour le flux particulaire, **caractérisé en ce que** le moyen de mesure est configuré pour évaluer au moins un quantile de la distribution de taille de particules à partir des au moins deux valeurs de mesure du résonateur à microondes.

10. Dispositif selon la revendication 9, **caractérisé en ce que** le moyen de mesure est disposé de telle façon qu'une température du flux particulaire est également mesurée.

11. Dispositif selon la revendication 9 ou 10, **caractérisé en ce que** le moyen de mesure est disposé de telle façon que les grandeurs caractéristiques sont mesurées dans une couche fluidisée.

12. Dispositif selon la revendication 11, **caractérisé en ce que** le moyen de mesure est configuré pour évaluer en outre l'une au moins des grandeurs suivantes telles que la quantité d'air alimenté et quantité de remplissage de la couche fluidisée.

13. Dispositif selon la revendication 11 ou 12, **caractérisé en ce que** le moyen de mesure est configuré pour approximer linéairement le quantile par les grandeurs de mesure évaluées.

14. Dispositif selon l'une des revendications 9 à 13, **caractérisé en ce que** le moyen de mesure est configuré pour déterminer le quantile pour une somme de distribution numérique, une somme de distribution des longueurs, une somme de distribution des surfaces ou une somme de distribution des volumes ou à une somme de distribution des masses.

15. Dispositif selon l'une des revendications 9 à 14, **caractérisé en ce que** le moyen de mesure est configuré pour détecter plusieurs quantiles dans le temps.

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009030314 A
- DE 10111833 C1
- DE 3241544 A1
- JP 4230058 B

**In der Beschreibung aufgeführte Nicht-Patentliteratur**

- **CLARK**. Particle size characterisation of metals powders for Additive Manufacturing using a microwave sensor. *POWDER TECHNOLOGY*, 02 December 2017, vol. 327, 536-543